(19) Europäisches Patentamt

European Patent Office

Office européen des brevets

(11) EP 1 579 802 A1

(12) EUROPEAN PATENT APPLICATION

(43) Date of publication:
28.09.2005 Bulletin 2005/39

(51) Int Cl.7: A61B 5/024

(21) Application number: 05251670.5

(22) Date of filing: 18.03.2005

(84) Designated Contracting States:
AT BE BG CH CY CZ DE DK EE ES FI FR GB GR HU IE IS IT LI LT LU MC NL PL PT RO SE SI SK TR
Designated Extension States:
AL BA HR LV MK YU

(30) Priority: 23.03.2004 KR 2004019626

(71) Applicant: SAMSUNG ELECTRONICS CO., LTD.
Gyeonggi-do (KR)

(72) Inventors:
• Lee, Hyoungki 401-1902 Cheonmyeong Maeul
Yeongtong-gu, Suwon-si, Gyeonggi-do (KR)

• Bang, Seokwon
718 Ilwon-dong Gangnam-gu Seoul (KR)
• Choi, Kiwan
Manan-gu Anyang-si Gyeonggi-do (KR)

(74) Representative: Ertl, Nicholas Justin
Elkington and Fife LLP,
Prospect House,
8 Pembroke Road
Sevenoaks,
Kent TN13 1XR (GB)

(54) **Method of and apparatus for measuring heart rate, and recording medium storing computer program for executing the method**

(57) There are provided a method of and an apparatus for measuring heart rate from blood flow in a blood vessel of a human body part, and a recording medium storing a computer program for executing the method. The apparatus comprises an amplifying circuit receiving a light signal reflected or penetrated from a human body part and generating a multi-stage amplified signal composed of signals having a plurality of different gains according to a control signal; and a processor separating the multi-stage amplified signal provided from the amplifying circuit into a plurality of channel signals having corresponding gains, reconfiguring the light signal using a channel signal selected among the plurality of channel signals and calculating heart rate from the reconfigured light signal. According to the apparatus and method, it is possible to make an accurate measurement of heart rate by minimizing effects of measuring environments. In addition, the apparatus and the method are resistive to noise resulting from a movement of a human body part by comparing an optimum signal pattern and a correlation coefficient.

FIG. 2

**Description**

BACKGROUND OF THE INVENTION

The present invention relates to a method of and an apparatus for measuring heart rate, and more particularly, to a method of and an apparatus for measuring heart rate by measuring a blood flow.

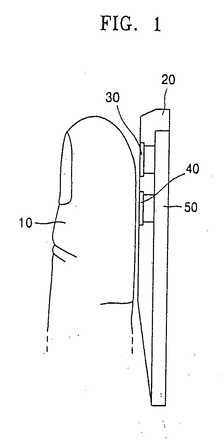

FIG. 1 shows an apparatus 20 for measuring heart rate by using blood flow. In general, the apparatus 20 measures the blood flow, oxygen saturation, or heart rate in a human body part such as finger, wrist, cheek, or earlobe by using a photoplethysmography (PPG) sensor.

The apparatus 20 comprises an optical transmitting circuit 30, an optical receiving circuit 40, and a processor 50. When infrared or visible light output from the optical transmitting circuit 30 is reflected from a blood vessel of a finger 10, the optical receiving circuit 40 detects an amplitude of the reflected light from the blood vessel of the finger 10. The amplitude of the reflected light depends on the blood flow in the blood vessel. The processor 50 measures heart rate using the reflected light detected by the optical receiving circuit 40.

However, measurement of the heart rate is often affected by a varying contact pressure between the finger 10 and the apparatus 20, a condition of a capillary vessel in the finger 10, or noise resulting from a movement of the finger 10, which results in deteriorating an accuracy in measurement.

SUMMARY OF THE INVENTION

According to an aspect of the present invention, there is provided an apparatus for measuring heart rate, comprising: an amplifying circuit receiving a light signal reflected or penetrated from a human body part and generating a multi-stage amplified signal composed of signals having a plurality of different gains according to a control signal; and a processor separating the multi-stage amplified signal provided from the amplifying circuit into a plurality of channel signals having corresponding gains, reconfiguring the light signal using a channel signal selected among the plurality of channel signals and calculating heart rate from the reconfigured light signal.

The amplifying circuit may comprise: an amplifier amplifying a difference between a signal input to a first input terminal and a signal input to a second input terminal; a plurality of resistors having different resistances; and a multiplexer transmitting the received light signal to a corresponding resistor among the plurality of resistors according to the control signal output from the processor, and wherein each of the plurality of resistors is connected between the first input terminal and the multiplexer.

The processor may comprise a first signal reconfiguring circuit separating the multi-stage amplified signal provided from the amplifying circuit into a plurality of channel signals having corresponding gains, dividing each of the plurality of channel signals into a unit of segments based on peak values of each of the plurality of channel signals, and generating a first reconfigured light signal including a channel signal selected among the plurality of channel signals with regard to each segment; a second signal reconfiguring circuit calculating a correlation coefficient from the first reconfigured signal and the basic pattern and generating a second reconfigured light signal using the correlation coefficient; and a heart rate calculating circuit comparing the second reconfigured signal with a reference correlation coefficient, and calculating the heart rate based on a signal obtained from the comparison result.

The processor may comprise a signal reconfiguring circuit separating the multi-stage amplified signal provided from the amplifying circuit into a plurality of channel signals having corresponding gains, dividing each of the plurality of channel signals into a unit of segments based on peak values of each of the plurality of channel signals, and generating the reconfigured light signal including a channel signal selected among the plurality of channel signals with regard to each segment; and a heart rate calculating circuit comparing the reconfigured light signal with a reference correlation coefficient, and calculating the heart rate based on a signal obtained from the comparison result.

According to another aspect of the present invention, there is provided an apparatus for measuring heart rate, comprising: a detecting circuit detecting a light signal reflected or penetrated from a human body part; and a processor receiving the light signal from the detecting circuit, generating a reconfigured signal based on a correlation between the light signal and a basic pattern, and calculating heart rate from the reconfigured signal.

The processor may comprise a signal reconfiguring circuit calculating a correlation coefficient from the light signal and the basic pattern and generating the reconfigured signal using the correlation coefficient; and a heart rate calculating circuit comparing the reconfigured signal with a reference correlation coefficient, and calculating the heart rate based on a signal obtained from the comparison result.

According to another aspect of the present invention, there is provided a method of measuring heart rate, comprising: receiving a light signal reflected or penetrated from a human body part; generating a multi-stage amplified signal by amplifying the light signal with a plurality of different gains according to a control signal; separating the multi-stage amplified signal into a plurality of channel signals having corresponding gains; reconfiguring the light signal using a channel signal selected among the plurality of channel signals; and calculating heart rate from the reconfigured light signal.

The reconfiguring the light signal comprises: dividing each of the plurality of channel signals in a unit of segments, and generating a first reconfigured light signal including a channel signal selected among the plurality of channel signals with regard to each segment; and generating a second reconfigured light signal based on a correlation between the first reconfigured signal and a basic pattern.

The reconfiguring the light signal comprises: dividing each of the plurality of channel signals in a unit of segments, and generating a reconfigured signal including a channel signal selected among the plurality of channel signals with regard to each segment.

A program for executing the method of measuring heart rate from blood flow in the blood vessel of the body part may be stored on a computer-readable recording medium.

The present invention thus provides a method of and an apparatus for measuring heart rate accurately by using blood flow, irrespective of a varying contact pressure between a human body part and the apparatus, a condition of a capillary vessel in the body part, or noise resulting from a movement of the body part.

BRIEF DESCRIPTION OF THE DRAWINGS

The above and other features and advantages of the present invention will become more apparent by describing in detail exemplary embodiments thereof with reference to the attached drawings in which:

FIG. 1 is a diagram showing a method of using an apparatus for measuring heart rate by using blood flow;
FIG. 2 is a block diagram showing an apparatus for measuring heart rate by using blood flow according to an embodiment of the present invention;
FIG. 3 is a detailed circuit diagram showing an amplifying circuit in FIG. 2;
FIG. 4 is a diagram showing input and output waveforms of a first signal reconfiguring circuit in FIG. 2;
FIG. 5 is a diagram showing an output waveform of a first signal reconfiguring circuit in FIG. 2;
FIG. 6 is a diagram showing an embodiment of a basic pattern of a waveform used for calculating a correlation coefficient in a second signal reconfiguring circuit in FIG. 2;
FIG. 7 is a diagram showing an output waveform of a first signal reconfiguring circuit including noise; and
FIG. 8 is a diagram showing a correlation coefficient of the output waveform in FIG. 7.

DESCRIPTION OF THE PREFERRED
EMBODIMENTS

Exemplary embodiments according to the present invention will now be described in detail with reference to the accompanying drawings. Like reference numerals in the drawings denote like elements.

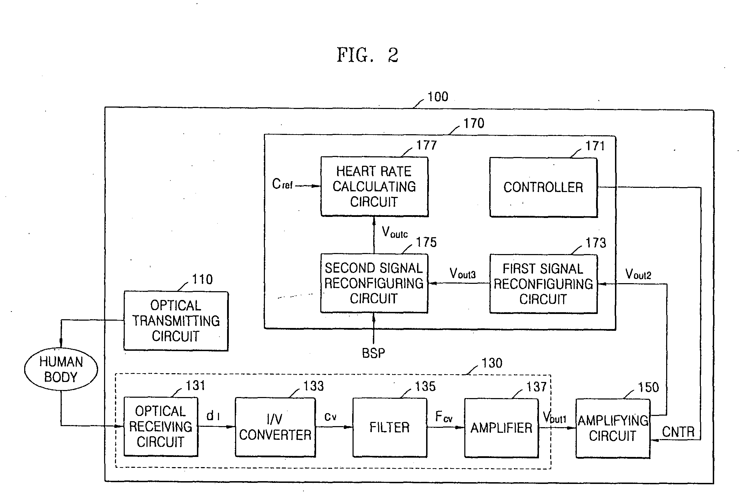

FIG. 2 is a block diagram showing an apparatus 100 for measuring heart rate by using blood flow according to an embodiment of the present invention. The apparatus 100 can be implemented with semiconductor chips. The apparatus 100 comprises an optical transmitting circuit 110, a detecting circuit 130, an amplifying circuit 150, and a processor 170.

The optical transmitting circuit 110 generates a light signal and outputs the light signal to a human body part. Examples of the light signal include infrared or visible light, but are not limited thereto.

The detecting circuit 130 receives the infrared or visible light reflected or penetrated from the body part, particularly a blood vessel of the body part, filters the received light signal, amplifies the filtered light signal, and outputs the amplified light signal $V_{out1}$ to an amplifying circuit 150. The detecting circuit 130 comprises an optical receiving circuit 131, an IN converter 133, a filter 135, and an amplifier 137.

The optical receiving circuit 131 detects an amplitude of the infrared or visible light reflected or penetrated from the body part, and generates a current $d_i$ according to the detected amplitude. The amplitude of the infrared or visible light received by the optical receiving circuit 131 varies according to a thickness of a blood vessel of the body part. For instance, when the infrared or visible light output from the optical transmitting circuit 110 is input to the body part during expansion of the blood vessel of the body part, the blood vessel of the body part absorbs plenty of the infrared or visible light. Thus, the amplitude of the infrared or visible light reflected or penetrated from the body part is reduced, whereby the amount of current output from the optical receiving circuit 131 is small.

The IN converter 133 receives the current $d_i$ from the optical receiving circuit 131 and generates a voltage $c_v$ corresponding to the current $d_i$. In other words, the IN converter 133 acts as a current/voltage converter.

The filter 135 is preferably implemented with a lowpass filter. The filter 135 receives the voltage $C_v$ from the IN converter 133, removes a high-frequency noise from the voltage $c_v$, and outputs the result $F_{cv}$. The amplifier 137 receives the voltage $F_{cv}$ output from the filter 135, amplifies the voltage $F_{cv}$, and outputs the result $V_{out1}$.

The amplifying circuit 150 generates a multi-stage amplified signal $V_{out2}$ composed of a sum of signals having "n" different gains in response to a control signal CNTR output from a controller 171 of the processor 170.

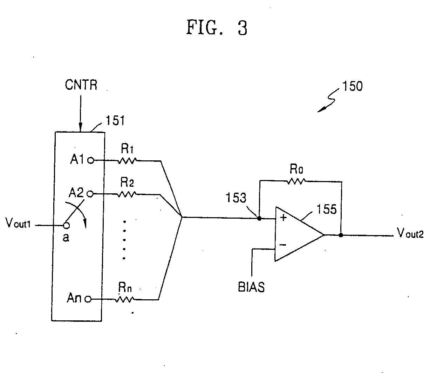

FIG. 3 shows a detailed circuit diagram of an amplifying circuit 150 in FIG. 2. The amplifying circuit 150 comprises an n-channel multiplexer 151, a plurality of resistors $R_1$ through $R_n$, a comparator 155, and a resistor $R_0$.

The n-channel multiplexer 151 transmits a voltage $V_{out1}$ output from the amplifier 137 to each of corresponding terminals A1 through An according to the control signal CNTR. Each of the resistors $R_1$ through $R_n$ is connected between each of the corresponding terminals A1 through An and a node 153. The resistances of the individual resistors $R_1$ through $R_n$ are preferably different from one another. A pair of each of the terminals A1 through An and each of the resistors $R_1$ through $R_n$ constitute a channel.

The resistor $R_0$ is connected between an input terminal 153 and an output terminal of the comparator 155. The comparator 155 receives a bias voltage BIAS and a voltage of the node 153, amplifies a difference between the received voltages, and outputs the result $V_{out2}$. The comparator 155 and the resistor $R_0$ may be replaced with a single OP amp having a feedback resistor $R_0$.

For instance, when the voltage $V_{out1}$ output from the amplifier 137 is input to the comparator 155 through the terminal A1 and the resistor $R_1$ according to the control signal CNTR, a voltage gain of the amplifying circuit 150 is proportional to $-(R_0/R_1)$. In addition, when the voltage $V_{out1}$ output from the amplifier 137 is input to the comparator 155 through the terminal An and the resistor $R_n$ according to the control signal CNTR, a voltage gain of the amplifying circuit 150 is proportional to $-(R_0/R_n)$. Therefore, the voltage gain of the amplifying circuit 150 varies like Equation 1 according to the control signal CNTR.

[Equation 1]

$$Gain = -\frac{R_0}{R_n}$$

Here, "n" denotes a natural number.

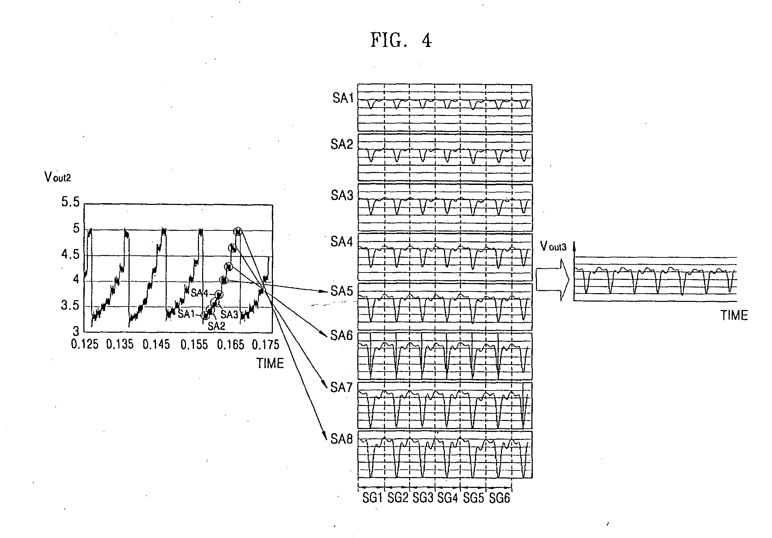

FIG. 4 shows input and output waveforms of a first signal reconfiguring circuit 173 in FIG. 2. Referring to FIGS. 3 and 4, the output voltage $V_{out2}$ of the amplifying circuit 150 is a signal obtained by superimposing a plurality of signals SA1 through SA8 having different gains. For convenience' sake, FIG. 4 shows an output signal $V_{out2}$ of the amplifying circuit 150 composed of an eight-channel multiplexer 151. In other words, the number of resistors and terminals in FIG. 3 is 8, respectively.

When a terminal "a" is sequentially connected to the terminals A1 through An (n=8) in each cycle, the output signal $V_{out2}$ of the amplifying circuit 150 is shown in FIG. 4. Therefore, the output signal $V_{out2}$ of the amplifying circuit 150 in each cycle is obtained by superimposing (or summing) signals having "n" different gains according to Equation 1.

The output signal $V_{out2}$ of the amplifying circuit 150 is separated into signals SA1 through SA8. SA1 represents the output signal $V_{out2}$ of the amplifying circuit 150 when the voltage $V_{out1}$ output from the amplifier 137 is input to the comparator 155 through the terminal A1 and the resistor R1. SAn (where "n" is 2 through 8) represents the output signal $V_{out2}$ of the amplifying circuit 150 when the voltage $V_{out1}$ output from the amplifier 137 is input to the comparator 155 through the terminal An (where "n" is 2 through 8) and the resistor $R_n$ (where "n" is 2 through 8).

The processor 170 comprises a controller 171, a first signal reconfiguring circuit 173, a second signal reconfiguring circuit 175, and a heart rate calculating circuit 177. The processor 170 may be implemented with semiconductor chips. In another embodiment, the processor 170 comprises a controller 171, a first signal reconfiguring circuit 173, and a heart rate calculating circuit 177. According to this, the output signal $V_{out3}$ of the first signal reconfiguring circuit 173 is directly provided to the heart rate calculating circuit 177. In still another embodiment, the processor 170 comprises a controller 171, a second signal reconfiguring circuit 175, and a heart rate calculating circuit 177. According to this, the output signal $V_{out1}$ of the amplifier 137 is directly provided to the second signal reconfiguring circuit 175.

The controller 171 provides the control signal CNTR to the amplifying circuit 150 for controlling an operation of the n-channel multiplexer 151.

The first signal reconfiguring circuit 173 receives the output signal $V_{out2}$ of the amplifying circuit 150, stores the received signals, detects peak values of the stored output signal $V_{out2}$, which are peak values of heart rate signals, and divides adjacent peak values in a unit of segments. The first signal reconfiguring circuit 173 selects a single signal having an optimum amplitude in each segment (SG1 through SG6 in FIG. 4), and generates a first reconfigured signal $V_{out3}$ including the selected signals. Each of the selected signals has an unsaturated highest peak value in each segment (SG1 through SG6 in FIG. 4). In addition, since the signals of the individual segments may be amplified with different gains, each of the signals selected in the individual segments is normalized by dividing by its gain, when the first reconfigured signal is generated.

For instance, assuming that a signal SA6 is selected in a segment SG1, a signal SA7 is selected in a segment SG2, a signal SA5 is selected in a segment SG3, a signal SA4 is selected in a segment SG4, a signal SA8 is selected in a segment SG5, and a signal SA1 is selected in a segment SG6, the first signal reconfiguring circuit 173 normalizes each of the signals selected in the segments SG1 through SG6, and outputs a reconfigured signal $V_{out3}$ resulting from the normalization.

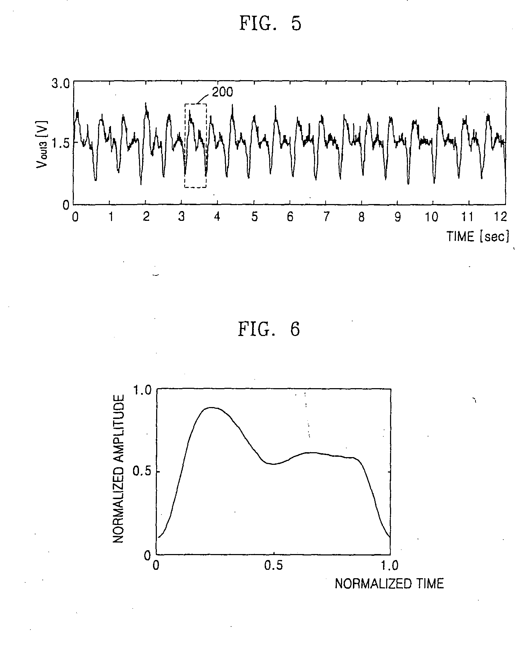

The second signal reconfiguring circuit 175 receives the output signal $V_{out3}$ from the first signal reconfiguring circuit 173 in FIG. 5, samples a signal 200 corresponding to one segment in the signal $V_{out3}$, normalizes the sampled signal 200, generates a basic pattern as shown in FIG. 6, and stores the basic pattern. In another embodiment, the second signal reconfiguring circuit 175 may receive and store the basic pattern BSP as shown in FIG. 6 from outside. The second signal reconfiguring circuit 175 receives the basic pattern BSP and the output signal $V_{out3}$ of the first signal reconfiguring circuit 173, estimates a correlation coefficient C between the basic pattern BSP and the signal of each segment in the output signal $V_{out3}$, and outputs a second, reconfigured signal $V_{outc}$ having the estimated correlation coefficient C in each segment to the heart rate calculating circuit 177.

The correlation coefficient C may be estimated through Equation 2.

[Equation 2]

$$C = \frac{\frac{1}{N+1} \sum_{i=0}^{N} (x_{k(i)} - \bar{x})(b_i - \bar{b})}{\sigma_x \sigma_b},$$

$$k(i) = \frac{(N - i)P_n + iP_{n+1}}{N}$$

where $X_{k(i)}$ denotes a k(i)-th signal, $b_i$ denotes an i-th basic pattern having a single cycle 0 through N, $\sigma_x$ and $\sigma_b$ denote standard deviations of x and b, respectively, $\bar{x}$ and $\bar{b}$ denote mean values of x and b, respectively, $P_n$ denotes a n-th peak point, $P_{n+1}$ denotes a (n+1)-th peak point, and i denotes a natural number.

Therefore, each of the segments SG1 through SG6 is a sector between a central point of a n-th peak point and a (n+1)-th peak point and a central point of a (n+1)-th peak point and a (n+2)-th peak point. However, the sector of each of the segments according to the present invention is not limited to the sector as defined above.

The first signal reconfiguring circuit 173 separates the output signal $V_{out2}$ of the amplifying circuit 150 according to channels, detects a peak value of a signal in each of the channels, allots a sector between neighboring peak values to a segment, selects signals allotted to each segment according to a predetermined standard, reconfigures the selected signals, and outputs the result $V_{out3}$. The predetermined standard is a standard for obtaining a signal having an unsaturated highest peak value.

The heart rate calculating circuit 177 receives the second reconfigured signal $V_{outc}$ having the correlation coefficient C and a reference correlation coefficient $C_{ref}$, compares the correlation coefficient C and the reference correlation coefficient $C_{ref}$, and calculates the heart rate from a signal having the correlation coefficient C larger than the reference correlation coefficient $C_{ref}$, according to the comparison result. The reference correlation coefficient $C_{ref}$ may be input from outside the apparatus 100, or be already stored in a memory of the heart rate calculating circuit 177. For instance, when the correlation coefficient C has a range of 0 through 1, the correlation coefficient C may be close to 1 for a normal signal, and the correlation coefficient C may be close to 0 for a signal with noise. However, a range of the correlation coefficient C may be diversely set.

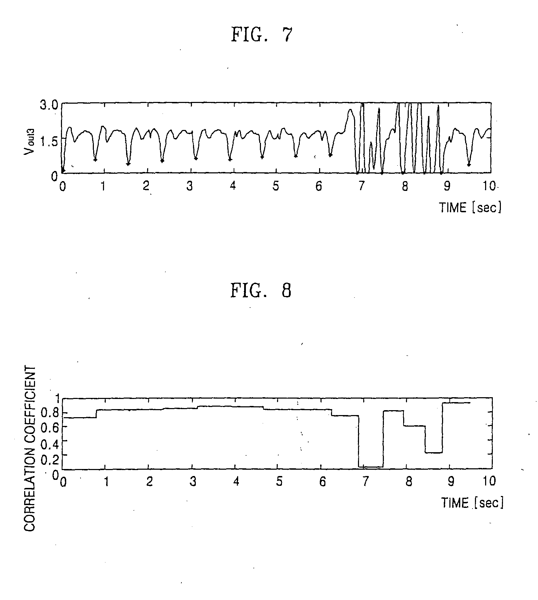

FIG. 7 shows an output waveform of a first signal reconfiguring circuit 173 including noise. FIG. 8 shows a correlation coefficient of the output waveform in FIG. 7.

Referring FIGS. 2, 7, and 8, when noise is included in the output signal $V_{out3}$ of the first signal reconfiguring circuit 173, the correlation coefficient C is close to 0. Therefore, the heart rate calculating circuit 177 counts only a signal having the correlation coefficient C greater than the reference correlation coefficient $C_{ref}$ among the output signal $V_{outc}$ of the second signal reconfiguring circuit 175, but does not count different signals regarded as noise. Therefore, even though noise is included in the output signal $V_{out3}$ of the first signal reconfiguring circuit 173, the heart rate calculating circuit 177 counts the heart rate excluding the noise by using the correlation, and outputs the result to a display (not shown).

Therefore, a method of measuring the heart rate by using the blood flow in a blood vessel of the body part can be used by the apparatus 100. In addition, the method may be implemented as a software program stored on a computer-readable recording medium. The computer-readable recording medium is any data storage device that can store data which can be thereafter read by a computer system. Examples of the computer-readable recording medium include read-only memory (ROM), random-access memory (RAM), CD-ROMs, magnetic tapes, floppy disks, optical data storage devices, and carrier waves (such as data transmission through the Internet). The computer-readable recording medium can also be distributed over network-coupled computer systems so that the computer-readable code is stored and executed in a distributed fashion. Also, functional programs, codes, and code segments for accomplishing the present invention can be easily construed by programmers skilled in the art to which the present invention pertains.

The method and apparatus according to the present invention can be used in a portable heart rate measuring instrument capable of measuring heart rate by using blood flow, an MP3 player having a function of measuring the amount of exercise, a stress measuring instrument, a calorie consumption measuring instrument or the like.

According to the present invention, since the method and apparatus for measuring heart rate by using blood flow does not require an additional operation of regulating a gain, it is possible to reduce the time for measuring the heart rate.

In addition, the method and apparatus for measuring heart rate by using blood flow is resistive to noise resulting from a movement of a body part by comparing an optimum signal pattern with a correlation coefficient.

In addition, the apparatus and method for measuring heart rate by using blood flow can prevent performance degradation resulting from a varying contact pressure between the body part and the apparatus.

While the present invention has been described with reference to exemplary embodiments thereof, it will be understood by those skilled in the art that various changes in form and details may be made therein without departing from the scope of the present invention as defined by the following claims.

**Claims**

1. An apparatus for measuring heart rate, comprising:

   an amplifying circuit receiving a light signal reflected or penetrated from a human body part and generating a multi-stage amplified signal composed of signals having a plurality of different gains according to a control signal; and a processor separating the multi-stage amplified signal provided from the amplifying circuit into a plurality of channel signals having corresponding gains, reconfiguring the light signal using a channel signal selected among the plurality of channel signals and calculating heart rate from the reconfigured light signal.

2. The apparatus according to claim 1, wherein the light signal is infrared or visible light.

3. The apparatus according to claim 1 or 2, further comprising:

   an optical transmitting circuit outputting the light signal to the human body part; and a detecting circuit detecting the light signal reflected or penetrated from the human body part to be provided to the amplifying circuit.

4. The apparatus according to claim 3, wherein the detecting circuit comprises:

   an optical receiving circuit receiving the light signal and detecting an amplitude of the received light signal; a converter converting current output from the optical receiving circuit into voltage; and a filter filtering the voltage output from the converter.

5. The apparatus according to claim 4, wherein the detecting circuit further comprises an amplifier receiving an output signal of the filter and amplifying the received signal.

6. The apparatus according to any preceding claim 1, wherein the amplifying circuit comprises:

   an amplifier amplifying a difference between a signal input to a first input terminal and a signal input to a second input terminal; a plurality of resistors having different resistances; and a multiplexer transmitting the received light signal to a corresponding resistor among the plurality of resistors according to the control signal output from the processor, and

   wherein each of the plurality of resistors is connected between the first input terminal and the multiplexer.

7. The apparatus according to any preceding claim, wherein the processor comprises:

   a first signal reconfiguring circuit separating the multi-stage amplified signal provided from the amplifying circuit into a plurality of channel signals having corresponding gains, dividing each of the plurality of channel signals into a unit of segments based on peak values of each of the plurality of channel signals, and generating a first reconfigured light signal including a channel signal selected among the plurality of channel signals with regard to each segment; a second signal reconfiguring circuit calculating a correlation coefficient from the first reconfigured light signal and the basic pattern and generating a second reconfigured light signal using the correlation coefficient; and a heart rate calculating circuit comparing the second reconfigured light signal with a reference correlation coefficient, and calculating the heart rate based on a signal obtained from the comparison result.

8. The apparatus according to claim 7, wherein each of the segments is a sector between a central point of a i-th peak point and a (i+1)-th peak point and a central point of a (i+1)-th peak point and a (i+2)-th peak point.

9. The apparatus according to claim 7, wherein the selected signal has an unsaturated highest peak value in a corresponding segment.

10. The apparatus according to any one of claims 1 to 6, wherein the processor comprises:

    a signal reconfiguring circuit separating the multi-stage amplified signal provided from the amplifying circuit into a plurality of channel signals having corresponding gains, dividing each of the plurality of channel signals into a unit of segments based on peak values of each of the plurality of channel signals, and generating the reconfigured light signal including a channel signal selected among the plurality of channel signals with regard to each segment; and a heart rate calculating circuit comparing the reconfigured signal with a reference correlation coefficient, and calculating the heart rate based on a signal obtained from the comparison result.

11. The apparatus according to claim 10, wherein each of the segments is a sector between a central point of a i-th peak point and a (i+1)-th peak point and a central point of a (i+1)-th peak point and a (i+2)-th peak point.

12. The apparatus according to claim 10, wherein the selected signal has an unsaturated highest peak value in a corresponding segment.

13. An apparatus for measuring heart rate, comprising:

a detecting circuit detecting a light signal reflected or penetrated from a human body part; and
a processor receiving the light signal from the detecting circuit, generating a reconfigured signal based on a correlation between the light signal and a basic pattern, and calculating heart rate from the reconfigured signal.

14. The apparatus according to claim 13, wherein the processor comprises:

a signal reconfiguring circuit calculating a correlation coefficient from the light signal and the basic pattern and generating the reconfigured signal using the correlation coefficient; and
a heart rate calculating circuit comparing the reconfigured signal with a reference correlation coefficient, and calculating the heart rate based on a signal obtained from the comparison result.

15. A method of measuring heart rate, comprising:

receiving a light signal reflected or penetrated from a human body part;
generating a multi-stage amplified signal by amplifying the light signal with a plurality of different gains according to a control signal;
separating the multi-stage amplified signal into a plurality of channel signals having corresponding gains;
reconfiguring the light signal using a channel signal selected among the plurality of channel signals; and
calculating heart rate from the reconfigured light signal.

16. The method according to claim 15, wherein the generating a multi-stage amplified signal comprises:

transmitting the light signal to a first input terminal of an amplifier through a corresponding channel among a plurality of channels according to the control signal; and
amplifying a difference between a signal input to the first input terminal and a signal input to a second input terminal.

17. The method according to claim 15 or 16, wherein the reconfiguring the light signal comprises:

dividing each of the plurality of channel signals in a unit of segments, and generating a first reconfigured light signal including a channel signal selected among the plurality of channel signals with regard to each segment; and
generating a second reconfigured light signal based on a correlation between the first reconfigured signal and a basic pattern.

18. The method according to claim 15 or 16, wherein the reconfiguring the light signal comprises:

dividing each of the plurality of channel signals in a unit of segments, and generating a reconfigured signal including a channel signal selected among the plurality of channel signals with regard to each segment.

19. A method of measuring heart rate, comprising:

receiving a light signal reflected or penetrated from a human body part;
generating a reconfigured light signal based on a correlation between the light signal and a basic pattern; and
calculating heart rate from the reconfigured signal.

20. A computer-readable recoding medium having recorded thereon a program for executing a method of measuring heart rate, comprising:

receiving a light signal reflected or penetrated from a human body part;
generating a multi-stage amplified signal by amplifying the light signal with a plurality of different gains according to a control signal;
separating the multi-stage amplified signal provided from the amplifying circuit into a plurality of channel signals having corresponding gains, dividing each of the plurality of channel signals in a unit of segments, and generating a first reconfigured signal including a channel signal selected among the plurality of channel signals with regard to each segment;
generating a second reconfigured signal based on a correlation between the first reconfigured signal and a basic pattern; and
calculating heart rate from the second reconfigured signal.

21. A computer-readable recoding medium having recorded thereon a program for executing a method of measuring heart rate, comprising:

receiving a light signal reflected or penetrated from a human body part;
generating a multi-stage amplified signal by amplifying the light signal with a plurality of different gains according to a control signal;
separating the multi-stage amplified signal provided from the amplifying circuit into a plurality of channel signals having corresponding gains, dividing each of the plurality of channel signals in a unit of segments, and generating a reconfigured signal including a channel signal selected among the plurality of channel signals with regard to each segment; and
calculating heart rate from the reconfigured signal.

22. A computer-readable recoding medium having recorded thereon a program for executing a method of measuring heart rate, comprising:

receiving a light signal reflected or penetrated from a human body part;
generating a reconfigured signal based on a correlation between the light signal and a basic pattern; and
calculating heart rate from the reconfigured signal.

FIG. 1

FIG. 2

FIG. 3

FIG. 4

EP 1 579 802 A1

FIG. 5

FIG. 6

FIG. 7

FIG. 8

**European Patent Office**

**EUROPEAN SEARCH REPORT**

Application Number

EP 05 25 1670

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2002/045806 A1 (BAKER CLARK R ET AL) 18 April 2002 (2002-04-18) * figure 1.a * * figure 3 * *paragraph 0003, sentences 4-6* *paragraph 0035, sentence 1* *paragraph 0056, sentences 7-11* *paragraph 0059, sentences 16-30* *paragraph 0143, sentences 21-22* | 13 | A61B5/024 |
| Y | * figure 1.a * | 14 | |
| A | * the whole document * | 1-12, 15-18 | |
| | ----- | | |
| X | EP 0 554 208 A (INCONTROL, INC) 4 August 1993 (1993-08-04) * column 11, line 20 - line 24 * * column 17, line 2 - line 5 * | 20-22 | |
| | ----- | | |
| Y | EP 1 393 673 A (PIONEER CORPORATION) 3 March 2004 (2004-03-03) * paragraph [0085] - paragraph [0087] * * paragraph [0092] * * paragraph [0093] * * paragraph [0095] * | 14 | TECHNICAL FIELDS SEARCHED (Int.Cl.7) |
| A | * the whole document * | 1-12, 15-18 | A61B |
| | ----- | | |
| A | EP 1 354 553 A (SAMSUNG ELECTRONICS CO., LTD) 22 October 2003 (2003-10-22) * the whole document * | 1-12, 15-18 | |
| | ----- | | |

The present search report has been drawn up for all claims

| Place of search | Date of completion of the search | Examiner |
|---|---|---|
| Munich | 2 August 2005 | Gentil, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 579 802 A1**

## ANNEX TO THE EUROPEAN SEARCH REPORT
## ON EUROPEAN PATENT APPLICATION NO.

EP 05 25 1670

This annex lists the patent family members relating to the patent documents cited in the above-mentioned European search report.
The members are as contained in the European Patent Office EDP file on
The European Patent Office is in no way liable for these particulars which are merely given for the purpose of information.

02-08-2005

| Patent document cited in search report | | Publication date | Patent family member(s) | | Publication date |
|---|---|---|---|---|---|
| US 2002045806 | A1 | 18-04-2002 | US | 6083172 A | 04-07-2000 |
| | | | US | 5853364 A | 29-12-1998 |
| | | | US | 2005143634 A1 | 30-06-2005 |
| | | | US | 2004158135 A1 | 12-08-2004 |
| | | | US | 2005085735 A1 | 21-04-2005 |
| | | | US | 2005124871 A1 | 09-06-2005 |
| | | | US | 2002137994 A1 | 26-09-2002 |
| | | | US | 6411833 B1 | 25-06-2002 |
| | | | US | 2004181134 A1 | 16-09-2004 |
| EP 0554208 | A | 04-08-1993 | US | 5313953 A | 24-05-1994 |
| | | | AT | 254878 T | 15-12-2003 |
| | | | AU | 659518 B2 | 18-05-1995 |
| | | | AU | 3044792 A | 15-07-1993 |
| | | | CA | 2086713 A1 | 15-07-1993 |
| | | | CA | 2177184 A1 | 15-07-1993 |
| | | | DE | 69333325 D1 | 08-01-2004 |
| | | | DE | 69333325 T2 | 26-08-2004 |
| | | | EP | 0554208 A2 | 04-08-1993 |
| | | | EP | 0941695 A2 | 15-09-1999 |
| | | | JP | 6000167 A | 11-01-1994 |
| | | | US | 5388578 A | 14-02-1995 |
| EP 1393673 | A | 03-03-2004 | JP | 2004089314 A | 25-03-2004 |
| | | | EP | 1393673 A1 | 03-03-2004 |
| | | | US | 2004044292 A1 | 04-03-2004 |
| EP 1354553 | A | 22-10-2003 | KR | 2003081903 A | 22-10-2003 |
| | | | EP | 1354553 A1 | 22-10-2003 |
| | | | JP | 2003310562 A | 05-11-2003 |
| | | | US | 2003212336 A1 | 13-11-2003 |

EPO FORM P0459

For more details about this annex : see Official Journal of the European Patent Office, No. 12/82

16